# FASCICULE DE BREVET EUROPEEN

(11) **EP 3 765 051 B1**
(45) Date de publication et mention de la délivrance du brevet: **09.06.2021**
(21) Numéro de dépôt: 19730397.7
(22) Date de dépôt: 07.05.2019
(51) Int. Cl.: A61K 36/87, A23K 10/30, A23K 50/75, A23K 50/80, A23K 20/121

(54) **AUGMENTATION DE LA TENEUR EN ACIDES GRAS ESSENTIELS DES OEUFS PAR LE BIAIS D'UNE SUPPLEMENTATION NUTRITIONNELLE DES ANIMAUX A L'AIDE D'UNE TRES FAIBLE DOSE D'UN EXTRAIT DE RAISIN RICHE EN FLAVONOIDES**
ERHÖHUNG DES GEHALTS AN ESSENZIELLEN FETTSÄUREN IN EIER DURCH TIERFUTTERERGÄNZUNG MIT SEHR NIEDRIEGEN VON FLAVONOID-REICHEN TRAUBENEXTRAKT
INCREASE OF ESSENTIAL FATTY ACIDS LEVELS IN EGGS BY FEED SUPPLEMENTATION WITH VERY LOW DOSE OF FLAVONOID-RICH GRAPE EXTRACT

(30) Priorité: 09.05.2018 FR 1870548
(43) Date de publication de la demande: 20.01.2021
(73) Titulaire: Nor-Feed, 49070 Beaucouzé (FR)
(72) Inventeur: ENGLER, Paul, 49140 La Chapelle Saint Laud (FR); GUILET, David, 49100 Angers (FR)
(74) Mandataire: Bringer IP
(86) Numéro de dépôt international: PCT/FR2019/051043
(87) Numéro de publication internationale: WO 2019/215415

(56) Documents cités:
- KARA KANBER ET AL: "Effects of grape pomace supplementation to laying hen diet on performance, egg quality, egg lipid peroxidation and some biochemical parameters", JOURNAL OF APPLIED ANIMAL RESEARCH, vol. 44, no. 1, janvier 2016 (2016-01), pages 303-310, XP002790293,
- Kangpanich Chanpim et al.: "Study on the Efficiency of Grape Seed Meals Used as Antioxidants in Layer Diets Enriched with Polyunsaturated Fatty Acids Compared with Vitamin E", Brazilian Journal of Poultry Science Brazilian Journal of Poultry Science, vol. 18, no. 4 1 décembre 2016 (2016-12-01), pages 655-662, XP002790294, Extrait de l'Internet: URL:https://www.researchgate.net/publicati on/313355147_Study_on_the_Efficiency_of_Gr ape_Seed_Meals_Used_as_Antioxidants_in_Lay er_Diets_Enriched_with_Polyunsaturated_Fat ty_Acids_Compared_with_Vitamin_E [extrait le 2019-04-03]
- MAGRONE THEA ET AL: "Administration of a Polyphenol-Enriched Feed to Farmed Sea Bass (Dicentrarchus labrax L.) Modulates Intestinal and Spleen Immune Responses", OXIDATIVE MEDICINE AND CELLULAR LONGEVITY, 2016, XP002790295,
- KANGPANICH CHANPIM ET AL: "Effects of arachidonic acid supplementation in maturation diet on female reproductive performance and larval quality of giant river prawn (Macrobrachium rosenbergii)", PEERJ, vol. 4, 29 novembre 2016 (2016-11-29), XP002790296,
- VIVEROS A ET AL: "Effects of dietary polyphenol-rich grape products on intestinal microflora and gut morphology in broiler chicks", POULTRY SCIENCE, vol. 90, no. 3, mars 2011 (2011-03), pages 566-578, XP002790297, ISSN: 0032-5791

## Description

La présente invention se rapporte au domaine de la nutrition d'animaux producteurs d'oeufs, plus particulièrement de la supplémentation alimentaire en vue de la reproduction et/ou de la ponte. Elle concerne tout particulièrement l'utilisation d'une composition alimentaire comprenant au moins un extrait de plante dans le but de modifier le profil lipidique des œufs, fécondés ou non, notamment d'augmenter leur proportion en au moins un acide gras polyinsaturé (AGPI), en particulier de la famille des des AGPI dit « oméga-3 ».

### Art antérieur

La reproduction représente une étape cruciale à la vie. Elle repose entre autres sur un fonctionnement optimal de la gamétogénèse pour la création de gamètes d'excellente qualité. Parmi les paramètres qualitatifs considérés, l'information génétique ainsi que les réserves nutritionnelles associées à ces gamètes représentent deux aspects essentiels.

Les matières grasses représentent une part fondamentale des réserves nutritionnelles utilisées pendant l'embryogénèse. Des études ont notamment démontré l'impact positif de certains acides gras, tels que les acides gras insaturés, en particulier sur le développement embryonnaire (Cherian, 2015).

La stratégie de modulation du profil lipidique des gamètes la plus commune consiste à supplémenter l'alimentation en matières premières riches en acides gras polyinsaturés (AGPIs)(Yannakopoulos, 2007). Cette technique induit cependant une plus grande susceptibilité de l'aliment à l'oxydation et nécessite l'addition d'antioxydants technologiques pour garantir son intégrité. Ces matières premières riches en AGPIs représentent également une portion importante de la ration alimentaire.

L'utilisation d'une solution alternative, permettant d'arriver aux mêmes fins (augmentation de la proportion d'AGPIs dans les réserves nutritionnelles des gamètes) par le biais d'ingrédients non sensibles à l'oxydation, et présents en plus faible quantité dans la ration (moins de 200mg/kg d'aliment complet) permettrait d'optimiser la formulation de la ration alimentaire et d'améliorer sa stabilité.

Il subsiste donc un besoin d'ingrédients non sensibles à l'oxydation et conduisant à moduler le profil lipidique des œufs et particulièrement à augmenter la proportion d'AGPIs dans les réserves nutritionnelles des œufs et plus particulièrement des gamètes.

Les polyphénols figurent parmi les substances très peu sensibles à l'oxydantion et sont connus pour leurs propriétés antioxydantes (Williamson & Manach, 2005). Des extraits de plantes riches en polyphénols sont utilisés en alimentation animale pour améliorer le statut antioxydant des animaux d'élevage. On retrouve parmi les extraits de plantes communément utilisées en alimentation animale, pour leur contenu en polyphénols, les extraits de raisin, de romarin, de thé ou encore de curcuma.

Le 21 février 2017, la Commission Européenne a publié un règlement autorisant l'utilisation d'un extrait de raisin comme additif pour l'alimentation animale au sein de l'Union Européenne (Règlement d'exécution 2017/307). Celui-ci comprend au moins 80 % de polyphénols totaux exprimés en équivalent catéchine, au moins 60% de proanthocyanidines, appartenant à la famille des flavonoïdes. Il contient également au moins 0,75% d'anthocyanes et d'anthocyanidines. La dose d'utilisation recommandée est de 5 à 100 grammes de l'extrait de raisin par tonne d'aliment complet distribué à l'animal ('Journal officiel L 44/2017', n.d.).

Les auteurs de la présente invention ont montré que l'utilisation d'au moins un extrait de raisin particulier permet de résoudre en tout ou partie les problématiques évoquées ci-dessus

### Résumé de l'invention

La présente invention a donc pour objet l'utilisation d'au moins un extrait de raisin, ledit au moins un extrait de raisin comprenant :
- de 50 à 95 % en poids de préférence 65 à 85% de polyphénols totaux, tout préférentiellement 80 à 85%, exprimés en équivalent catéchine, par rapport au poids total de l'extrait,
- de 30% à 90 % en poids de préférence 50 à 70% de proanthocyanidines, tout préférentiellement 60 à 70% par rapport au poids total de l'extrait,
- de 0.5 à 10% en poids de préférence 0.5 à 2%, tout préférentiellement 0.75 à 2% d'anthocyanes et anthocyanidines totales par rapport au poids total de l'extrait.
pour modifier le profil lipidique des œufs chez un animal dit supplémenté par ledit au moins un extrait de raisin, choisi dans le groupe formé par les ovipares, les ovovivipares, en particulier les poissons et les volailles.

On entend par « animal supplémenté » un animal ayant consommé la composition alimentaire comprenand ledit au moins un extrait de raisin selon l'invention.

On entend par « animal non supplémenté » un animal ayant consommé une composition alimentaire ne comprenant pas ledit au moins un extrait de raisin selon l'invention.

Comme déjà mentionné, ledit au moins un extrait de raisin utilisé selon l'invention est faiblement sensible à l'oxydation, voire non sensible à l'oxydation. Par conséquent il peut être utilisé pour l'obtention d'une composition alimentaire stable vis-à-vis de l'oxydation, sans addition d'agents anti-oxydants. Plus particulièrement, ledit au moins un extrait de raisin ne nécessite pas l'addition supplémentaire d'au moins un des composés choisis dans le groupe constitué par les antioxidydants technologiques, dont notament l'éthoxyquine, l'hydroxyanisole butylé (BHA) ou l'hydroxytoluène butylé (BHT).

En outre ledit au moins un extrait de raisin utilisé selon l'invention permet l'augmentation du taux d'au moins un acide gras polyinsaturé, en particulier d'au moins un acide gras polyinsaturé de la famille des acides gras polyinsaturés dit « oméga-3 » dans les œufs des animaux dont l'alimentation a été supplémentée au moyen de cet extrait, notamment l'acide alpha linolénique et/ou l'acide docoxahexaènoïque, en particulier l'acide docoxahexaènoïque, par rapport aux œufs des animaux non supplémentés par ledit au moins un extrait de raisin. L'utilisation dudit au moins un extrait de raisin selon l'invention induit notamment l'augmentation d'au moins 10% d'au moins un acide gras polyinsaturé, en particulier de la famille des acides gras polyinsaturés dit « oméga-3 », dans les œufs dudit animal supplémenté, de préférence d'au moins 15% et tout préférentiellement d'au moins 20% %, en poids par rapport au poids des acides gras polyinsaturés dans les œufs dudit animal non supplémenté par ledit au moins un extrait de raisin selon l'invention.

On entend par « acide gras polyinsaturé » un acide gras dont la chaine carbonée comprend plusieurs liaisons carbone-carbones insaturées.

On entend par « acide gras polyinsaturé de la famille des acides gras polyinsaturés dit « oméga-3 » » des acides gras dont la chaine carbonée comprend plusieurs liaisons carbone-carbones insaturées et dont la première double liaison de la chaîne carbonée de l'acide, en comptant depuis l'extrémité opposée au carboxyle, est située sur la troisième liaison carbone-carbone.

Un autre avantage de l'invention est qu'elle permet de supprimer ou au moins de réduire la quantité d'ingrédients classiquement utilisés dans les compositions alimentaires pour animaux tels que des agents anti-oxydants et des acide(s) gras polyinsaturé(s) et ainsi de diminuer les coûts de production et également l'impact environnemental de ces compositions.

Encore un autre avantage de l'invention est que l'extrait de raisin peut être utilisé dans la composition alimentaire selon l'invention dans des quantités généralement très faibles, c'est-à-dire dans des doses inférieures ou égales à 200ppm, de préférence inférieures ou égales à 100ppm, ce qui permet d'optimiser la formulation de la composition alimentaire.

D'autres objets, aspects, avantages, propriétés de la présente invention sont présentés dans la description détaillée et les exemples qui suivent.

### Description détaillée

### Extrait de raisin.

On entend par « extrait de raisin », un extrait issu du genre botanique *(Vitis),* et notamment le *Vitis vinifera* et/ou *Vitis labrusca* et/ou *Vitis riparia* et/ou *Vitis rupestris* et/ou *Vitis berlandieri* et/ou *Vitis amurensis* et/ou *Vitis coignetiae* et/ou *Vitis vulpina* et/ou *Vitis acerifolia* et/ou *Vitis aestivalis* et/ou *Vitis rotundifolia,* de préférence le *Vitis vinifera.*

Pour préparer l'extrait de raisin utilisé dans l'invention, un élément de la structure d'une plante notamment choisi dans le groupe formé par les baies, les pépins les pellicules ou leurs mélanges sont utilisés. De préférence, les pellicules et/ou pépins de raisins, de manière tout à fait préférée les pellicules et pépins de raisin sont utilisés.

Comme déjà mentionné, ledit au moins un extrait de raisin utilisable selon l'invention comprend :
de 50 à 95 % en poids de préférence 65 à 85% de polyphénols totaux, tout préférentiellement 80 à 85%, exprimés en équivalent catéchine, par rapport au poids total de l'extrait,
de 30% à 90 % en poids de préférence 50 à 70% de proanthocyanidines, tout préférentiellement 60 à 70% par rapport au poids total de l'extrait,
de 0.5 à 10% en poids de préférence 0.5 à 2%, tout préférentiellement 0.75 à 2% d'anthocyanes et anthocyanidines totales par rapport au poids total de l'extrait.

On entend par « polyphénols » des composés chimiques naturels, caractérisés par présence d'au moins deux groupes phénoliques associés en structures plus ou moins complexes. Ils comprennent entre autres entre autres constitués du sous-groupe des flavonoïdes qui regroupe notamment les proanthocyanidines, les anthocyanes et les anthocyanidines.

On entend par « proanthocyanidine » des polymères de catéchines. Ils sont constitués d'unités catéchines liées entre elles par des liaisons carbone-carbone de type 4→8 ou 4→6.

On entend par « anthocyanidine » des pigments naturels, basés sur la structure de l'ion flavylium (ion 2-phénylchroménylium).

On entend par « anthocyane » les dérivés hétérosides des anthocyanidines

### Procédé de préparation

De manière préférée, le procédé d'obtention de l'extrait de raisin tel que que décrit dans l'invention consiste à réaliser une extraction de raisin (baie entière de *Vitis vinifera* dépulpée) à l'aide de solvants non synthétiques suivie d'une purification, puis d'une atomisation de l'extrait, ou à mélanger de manière homogène au moins un extrait sec de pellicule de raisin, contenant au moins 1% d'anthocyanes et d'anthocyanidines totales, avec au moins un extrait sec de pépins de raisin, contenant au moins 1% de flavonoïdes totaux. Le procédé de fabrication résulte en une composition contenant :
- de 50 à 95 % en poids de préférence 65 à 85% de polyphénols totaux, tout préférentiellement 80 à 85%, exprimés en équivalent catéchine, par rapport au poids total de l'extrait,
- de 30% à 90 % en poids de préférence 50 à 70% de proanthocyanidines, tout préférentiellement 60 à 70% par rapport au poids total de l'extrait,
- et de 0.5 à 10% en poids de préférence 0.5 à 2%, tout préférentiellement 0.75 à 2% d'anthocyanes et anthocyanidines totales par rapport au poids total de l'extrait,
en particulier, de manière à obtenir un extrait conforme au règlement autorisant l'utilisation d'un extrait de raisin comme additif pour l'alimentation animale au sein de l'Union Européenne (Règlement d'exécution 2017/307).

Bien entendu, le procédé de fabrication doit de manière préférée respecter les bonnes pratiques de fabrication.

### Composition alimentaire

L'invention concerne également l'utilisation d'au moins un extrait de raison selon l'invention dans une composition alimentaire, ledit au moins un extrait de raisin étant inclus dans une composition alimentaire.

Avantageusement, l'utilisation de l'extrait de raisin selon l'invention ne nécessite pas d'addition supplémentaire d'antioxydant technologique dans la composition alimentaire, par rapport à une composition alimentaire non supplémentée par un extrait de raisin selon l'invention.

La composition alimentaire peut comprendre en outre un véhicule physiologiquement acceptable, conforme à une utilisation par voie orale.

Selon un premier mode de réalisation, la composition alimentaire est sous forme solide, de préférence elle est alors choisie dans le groupe formé par une poudre, un granulé, un comprimé, un pellet extrudé ou non.

Lorsque la composition alimentaire est sous forme solide, alors la quantité en extrait de raisin est comprise entre 1 et 200mg/kg de préférence entre 5 et 100 mg/kg dans la composition sèche, par conséquent la quantité en extrait de raisin va de 10-4 % à 2. 10-2 %, de préférence de 5.10-4 % à 10-2 % en poids par rapport au poids total de l'extrait sec de la composition alimentaire.

Selon un autre mode de réalisation, la composition alimentaire est sous forme liquide, de préférence elle est alors choisie dans le groupe formé par une suspension, une solution, une eau de boisson.

Lorsque la composition alimentaire est sous forme liquide, alors la quantité en extrait de raisin est compris entre 1 et 100mg/L, de préférence entre 2.5 et 50 mg/L, par conséquent la quantité en extrait de raisin va de 10-4 % à 10-2 % de préférence de 2,5.10-4 % à 5.10-1 % en poids par rapport au poids total de la composition alimentaire.

La composition alimentaire comprend donc une très faible dose d'extrait de raisin.

La composition alimentaire selon l'invention comprenant généralement au moins un excipient choisi dans le groupe formé par additifs zootechniques, les additifs technologiques.

La préparation de la composition alimentaire utilisée selon l'invention met en œuvre des procédés classiques qui font partie des compétences générales de l'homme du métier.

La composition alimentaire utilisée selon l'invention peut notamment être sous forme d'un additif, d'une matière première ou d'un aliment composé.

On entend par « aliment composé», un mélange d'au moins deux matières premières pour aliments des animaux, comprenant ou non des additifs pour l'alimentation animale, qui est destiné à l'alimentation animale par voie orale, sous la forme d'un aliment complet pour animaux ou d'un aliment complémentaire pour animaux (Règlement767/2009).

On entend par « matière première », tous produits d'origine végétale ou animale, à l'état naturel, frais ou conservés, et dérivés de leur transformation industrielle, ainsi que substances organiques ou inorganiques , comprenant ou non des additifs, qui sont destinés à être utilisés pour l'alimentation des animaux par voie orale, soit directement tels quels, soit après transformation, pour la préparation d'aliments composés pour animaux, ou en tant que supports des prémélanges (Directive96/25/CE du Conseil du 29 avril 1996).

La composition alimentaire selon la présente invention peut être utilisée directement en tant qu'aliment, on peut alors la considérer comme une « matière première » dans la préparation dudit aliment composé.

On entend par « additif », toutes substances, micro-organismes ou préparations, autres que les matières premières pour aliments des animaux et les prémélanges, délibérément ajoutés aux aliments pour animaux ou à l'eau pour remplir notamment une ou plusieurs des fonctions suivantes : répondre aux besoins nutritionnels des animaux, avoir un effet positif sur les caractéristiques des aliments pour animaux ou des produits d'origine animale, sur la couleur des poissons ou oiseaux d'ornement, sur les conséquences environnementales de la production animale, sur la production, le rendement ou le bien-être des animaux, ou avoir un effet coccidiostatique ou histomonostatique (Règlement EU 1831/2003).

### Utilisations

La présente invention concerne l'utilisation d'au moins un extrait de raison selon l'invention ou d'une composition alimentaire contenant au moins un extrait de raisin selon l'invention pour modifier le profil lipidique des œufs chez un animal choisi dans le groupe formé par les ovipares, les ovovivipares, en particulier les poissons et les volailles.

Par « ovipare » au sens de la présente invention, on entend un animal qui se reproduit par des œufs pondus avant ou après fécondation mais avant éclosion. Cette définition s'applique par exemple à un poisson qui pond des œufs dont la fécondation est externe. La femelle pond les oeufs qui sont fécondés dans l'eau par le mâle. La croissance embryonnaire se terminera hors de l'organisme maternel. Les œufs éclosent par la suite et le temps avant éclosion varie selon les poissons et la température.

Par « ovovivipare» au sens de la présente invention, on entend un animal qui se reproduit par des œufs mais qui les conserve dans ses voies génitales jusqu'à l'éclosion des jeunes, l'embryon se développant uniquement à partir des réserves accumulées dans l'œuf. Cette définition s'applique par exemple à un poisson qui porte des oeufs qui incubent et qui éclosent dans le ventre de la mère, sans relation nutritive avec celle-ci (simples échanges d'eau et de gaz). L'embryon ovovivipare se nourrit dans l'œuf et éclot juste avant la naissance.

Dans le cadre de la présente invention, ledit au moins un extrait de raisin est utilisée chez un animal adulte c'est-à-dire qui a atteint sa maturité sexuelle et est en capacité de se reproduire, de préférence pendant une période allant de 2 semaines à 8 mois.

Avantageusement, la modification du profil lipidique des œufs consiste à induire dans les œufs dudit animal dit supplémenté par ledit au moins un extrait de raisin selon l'invention, une augmentation du taux d'au moins un acide gras polyinsaturé, plus particulièrement du taux d'au moins un acide gras de la famille des acides gras polyinsaturés dit « oméga-3 » (par rapport aux œufs dudit animal non supplémenté par ledit au moins un extrait de raisin selon l'invention. Avantageusement, ledit au moins un acides gras polyinsaturé de la famille des oméga-3 (AGPIs) dont le taux est augmenté est l'acide alpha-linolénique (ALA) et/ou l'acide docosahexaenoïque (DHA), tout particulièrement l'acide docosahexaenoïque (DHA).

Généralement le taux d'au moins un acide gras polyinsaturé dans les œufs dudit animal supplémenté par ledit au moins un extrait de raisin selon l'invention est augmenté d'au moins 10%, de préférence d'au moins 15% et tout préférentiellement d'au moins 20%, en poids par rapport au poids des acides gras polyinsaturés dans les œufs dudit animal non supplémenté par ledit au moins un extrait de raisin.

Par conséquent, selon l'invention, en l'utilisation dans le régime alimentaire d'un animal producteurs d'œufs ledit au moins un extrait de raisin selon l'invention à une très faible dose, permet d'obtenir une augmentation significative du taux d'au moins un acide gras polyinsaturé dans les œufs dudit animal supplémenté, en particulier une augmentation significative du taux de ALA et/ou DHA, tout particulièrement du taux de DHA.

Selon une première variante, les œufs obtenus par la mise en œuvre de la présente invention sont non fécondés ; il s'agit d'œufs enrichis en acide(s) gras insaturé(s) destinés à la consommation humaine

Selon une deuxième variante, les œufs obtenus par la mise en œuvre de la présente invention sont fécondés ; il s'agit d'œufs destinés à la reproduction qui présentent des réserves nutritionnelles optimisés.

Les exemples qui suivent visent à illustrer l'invention sans en limiter la portée.

### EXEMPLES

Exemples de modes de réalisation de l'invention. Les exemples 1 et 2 présentent des modes de réalisation de l'invention chez deux espèces de poissons et l'exemple 3 présente un mode de réalisation de l'invention chez une espèce de volaille.

### Exemple 1 : Modulation du profil d'acide gras dans les œufs de truite arc-en-ciel (Oncorhynchus mykiss)

Deux aliments ont été testés, différant uniquement par la présence de 60ppm d'extrait de raisin dans l'aliment du groupe « supplémenté ». Les aliments étaient composés de farines de poissons, feveroles dépelliculée, gluten de blé, tourteau d'extraction de soja cuit, huiles de poissons, blé, gluten de maïs, huile de colza, tourteau de colza, minéraux, vitamines.

Composition des aliments des deux groupes.

**[Tableaux 1]**

| **Groupe** | **Contrôle (CTL)** | **Supplémenté (NG)** |
|---|---|---|
| Protéines (%) | 39 | 39 |
| Lipides (%) | 27 | 27 |
| Dont oméga-3 (en % des lipides) | 15.5 | 15.5 |
| Cellulose (%) | 1.7 | 1.7 |
| Cendres (%) | 7 | 7 |
| Extrait de raisin selon l'invention (mg/kg) | **0** | **60** |

Chaque groupe était composé de 130 géniteurs de truite arc-en-ciel, comprenant chacun deux bassins de 40 femelles et un bassin de 50 mâles. Les poissons du même sexe étaient répartis de manière aléatoire entre les deux groupes.

L'ensemble des bassins étaient approvisionnés par le même circuit d'eau et la qualité de celle-ci était maîtrisée et contrôlée quotidiennement.

L'essai de supplémentation s'est déroulé sur la période des 5 mois précédant la ponte.

Les œufs des femelles étaient ensuite récoltés puis conservés à -80°C jusqu'à analyse.

Dix échantillons d'œufs de chaque groupe étaient soumis au protocole de préparation suivant :
- Lyophilisation de 10g d'œufs frais,
- Extraction automatique sous pression de 2g de lyophilisat d'œufs à l'aide de dichlorométhane (DCM),
- Evaporation à sec des extraits liquides obtenus,
- Saponification de 50mg d'extrait sec
- Dérivatisation à l'aide de BSTFA (N,O-Bis-triméthylsilyl-trifluoroacétamide),
- Injection en GC-MS

L'aire sous la courbe des pics de DHA était ensuite intégrée automatiquement afin de permettre une comparaison statistique des résultats à l'aide du test statistique de t-test (Welch, logiciel R, v3.4.1).

Résultats de l'intégration des pics de DHA des oeufs de truite arc-en-ciel et analyse statistique associée.

**[Tableaux 2]**

| Groupe | CTL | NG |
|---|---|---|
| Aire sous la courbe du pic de DHA (mUA) | 32861606 | 40507107 |

Les résultats de l'analyse des œufs de truite arc-en-ciel ont donc démontré une augmentation significative de la proportion en poids de DHA dans les œufs des poissons supplémentés en extrait de raisin (+23.3%, p<0,05).

### Exemple 2 : Modulation du profil d'acides gras dans les œufs de mulet (Mugil cephalus)

Deux aliments étaient testés, différant par la présence de 100ppm d'extrait de raisin dans l'aliment du groupe supplémenté (« NG »). Les aliment étaient composés de farines de poisson, gluten de blé, tourteau d'extraction de soja cuit, gluten de maïs, huiles de poissons, vitamines, minéraux.

Composition des aliments des deux groupes.

**[Tableaux 3]**

| Groupe | Contrôle (CTL) | Supplémenté (NG) |
|---|---|---|
| Protéine (%) | 34.2 | 35.6 |
| Lipides (%) | 9.1 | 9.5 |
| Dont oméga-3 (en % des lipides) | 7.7 | 6.6 |
| Cendres (%) | 9.1 | 8.0 |
| Extrait de raisin selon l'invention (mg/kg) | **0** | **100** |

Chaque groupe était composé de 5000 mulets (non-sexés) sexuellement matures (3 ans) répartis de manière aléatoire dans des bassins d'élevage extérieurs.

Les poissons étaient nourris pendant 3 mois avant la récolte des ovaires.

A l'issue de la phase d'élevage, une analyse des profils d'acides gras était réalisée sur un mélange d'ovaires de 10 poissons par groupe selon le protocole suivant :
- Extraction de Folch (Folch et al. 1957)
- Saponification
- Transméthylation (Norme ISO 12966),
- Analyse en GC-MS.

Les résultats étaient ensuite intégrés automatiquement de manière à définir la proportion relative de différents acides gras dans le profil lipidique des ovaires de mulets.

Résultats de l'intégration des pics de DHA des oeufs de truite arc-en-ciel.

**[Tableaux 4]**

| | | |
|---|---|---|
| Groupe | CTL | NG |
| Proportion relative de DHA (% des acides gras totaux) | 5,71% | 6,52% |

Les résultats de l'analyse des ovaires de mulet ont donc démontré une augmentation de la proportion en poids de DHA dans les ovaires des poissons supplémentés en extrait de raisin (+14.2%).

### Exemple 3 : Modulation du profil d'acides gras dans les œufs de poule pondeuse (Gallus gallus domesticus)

Deux aliments étaient testés, différents uniquement par la présence de 30ppm d'extrait de raisin dans l'aliment du groupe supplémenté (« NG »). Les aliments étaient composés de blé, maïs, tourtaux de soja, tourtaux de tournesol, carbonate de calcium, tourtaux de colza, minéraux, vitamines, acides aminés.

Composition des aliments des deux groupes.

**[Tableaux 5]**

| Groupe | Contrôle (CTL) | Supplémenté (NG) |
|---|---|---|
| Protéine (%) | 15.5 | 15.5 |
| Lipides (%) | 2.9 | 2.9 |
| Cellulose (%) | 4.6 | 4.6 |
| Cendres (%) | 12.1 | 12.1 |
| Extrait de raisin selon l'invention (mg/kg) | 0 | 30 |

Chaque groupe était constitué de 286 poules, réparties en 11 cages de 26 poules, distribuées de manière aléatoire dans la même batterie.

La période de supplémentation était de 5 semaines d'alimentation. Quatre œufs par cage étaient récoltés le premier et le dernier jour de l'essai pour les deux groupes. Ils étaient alors soumis au protocole suivant :
- Séparation des jaunes,
- Homogénéisation de 4 jaunes d'œuf par cage,
- Lyophilisation des jaunes,
- Extraction de Folch (Folch et al., 1957) sur 200mg de lyophylisat
- Evaporation l'extrait liquide obtenu,
- Transméthylation de l'extrait sec (Norme ISO 12966),
- Analyse en GC-MS.

L'aire sous la courbe des pics de DHA était ensuite intégrée automatiquement afin de permettre une comparaison statistique des résultats à l'aide du test statistique de t-test (Welch, logiciel R, v3.4.1).

Résultats de l'intégration des pics de DHA des oeufs de truite arc-en-ciel et analyse statistique associée.

**[Tableaux 6]**

| | | |
|---|---|---|
| Groupe | CTL | NG |
| Aire sous la courbe du pic de DHA (mUA) | 28636531 | 34651862 |

Les résultats de l'analyse des œufs de poule ont donc montré une augmentation significative de la proportion en poids de DHA dans les œufs des volailles supplémentés en extrait de raisin (+21.0%, p<0,05).

### Références :

Cherian, G. (2015). Nutrition and metabolism in poultry: role of lipids in early diet. Journal of Animal Science and Biotechnology, 6, 28. https://doi.org/10.1186/s40104-015-0029-9

Folch, J., Lees, M., & Sloane Stanley, G. H. (1957). A simple method for the isolation and purification of total lipides from animal tissues. The Journal of Biological Chemistry, 226(1), 497-509.

Journal officiel L 44/2017. (n.d.). Retrieved 20 March 2018, from http://eur-lex.europa.eu/legal-content/FR/TXT/HTML/?uri=OJ:L:2017:044:FULL&from=FR

Williamson, G., & Manach, C. (2005). Bioavailability and bioefficacy of polyphenols in humans. II. Review of 93 intervention studies. The American Journal of Clinical Nutrition, 81(1), 243S-255S.

Yannakopoulos, A. L. (2007). Egg enrichment in omega-3 fatty acids. In Bioactive Egg Compounds (pp. 159-170). https://doi.org/10.1007/978-3-540-37885-3_20

## Revendications

1. Utilisation d'au moins un extrait de raisin, ledit au moins un extrait de raisin comprenant :
a) de 50 à 95 % en poids de préférence 65 à 85% de polyphénols totaux, tout préférentiellement 80 à 85%, exprimés en équivalent catéchine, par rapport au poids total de l'extrait,
b) de 30% à 90 % en poids de préférence 50 à 70% de proanthocyanidines, tout préférentiellement 60 à 70% par rapport au poids total de l'extrait,
c) de 0.5 à 10% en poids de préférence 0.5 à 2%, tout préférentiellement 0.75 à 2% d'anthocyanes et anthocyanidines totales par rapport au poids total de l'extrait.
pour modifier le profil lipidique des œufs chez un animal dit supplémenté par ledit au moins un extrait de raisin, choisi dans le groupe formé par les ovipares, les ovovivipares, en particulier les poissons et les volailles.

2. Utilisation d'au moins un extrait de raisin selon la revendication 1 pour augmenter dans les œufs dudit animal supplémenté le taux d'au moins un acide gras polyinsaturé, plus particulièrement le taux d'au moins un acide gras polyinsaturé de la famille des acides gras polyinsaturés dit « oméga-3 », par rapport aux œufs dudit animal non supplémenté par ledit au moins un extrait de raisin.

3. Utilisation d'au moins un extrait de raisin selon la revendication précédente, **caractérisée en ce que** ledit au moins un acide gras polyinsaturé est l'acide alpha-linolénique et/ou l'acide docosahexaènoïque, en particulier l'acide docosahexaènoïque.

4. Utilisation d'au moins un extrait de raisin selon l'une quelconque des revendications 1 à 3, **caractérisée en ce que** le taux d'au moins un acide gras polyinsaturé dans les œufs dudit animal supplémenté est augmenté d'au moins 10%, de préférence d'au moins 15% et tout préférentiellement d'au moins 20%, en poids par rapport au poids des acides gras polyinsaturés dans les œufs dudit animal non supplémenté par ledit au moins un extrait de raisin.

5. Utilisation d'au moins un extrait de raisin selon l'une quelconque des revendications 1 à 4, ledit au moins un extrait de raisin étant inclus dans une composition alimentaire, ladite composition alimentaire étant un additif, un aliment, une matière première ou un aliment complémentaire.

6. Utilisation d'au moins un extrait de raisin selon la revendication 5 **caractérisée en ce que** ladite composition alimentaire est sous forme solide.

7. Utilisation d'au moins un extrait de raisin selon la revendication 6 **caractérisée en ce que** la quantité en extrait de raisin va de 10-4 % à 2. 10-2 %, de préférence de 5.10-4 % à 10-2 % en poids par rapport au poids total de l'extrait sec de la composition alimentaire

8. Utilisation d'au moins un extrait de raisin selon la revendication 5 **caractérisée en ce que** la composition alimentaire est sous forme liquide.

9. Utilisation d'au moins un extrait de raisin selon la revendication 8 **caractérisée en ce que** la quantité en extrait de raisin va de 10-4 % à 10-2 % de préférence de 2,5.10-4 % à 5.10-1 % en poids par rapport au poids total de la composition alimentaire.

10. Utilisation d'au moins un extrait de raisin selon l'une quelconque des revendications 1 à 9 **caractérisée en ce que** les œufs sont destinés à la consommation humaine ou à la reproduction.

## Patentansprüche

1. Verwendung von mindestens einem Traubenextrakt, worin der mindestens eine Traubenextrakt umfasst:
a) 50 bis 95 Gew.-%, vorzugsweise 65 bis 85 Gew.-%, am meisten bevorzugt 80 bis 85 Gew.-% Gesamtpolyphenole, ausgedrückt als Catechin-Äquivalent, bezogen auf das Gesamtgewicht des Extrakts,
b) 30 bis 90 Gew.-%, vorzugsweise 50 bis 70 Gew.-%, am meisten bevorzugt 60 bis 70 Gew.-% Proanthocyanidine, bezogen auf das Gesamtgewicht des Extrakts,
c) 0,5 bis 10 Gew.-%, vorzugsweise 0,5 bis 2 Gew.-%, am meisten bevorzugt 0,75 bis 2 Gew.-% Gesamtanthocyanine und Anthocyanidine, bezogen auf das Gesamtgewicht des Extrakts,
zum Zweck der Modifizierung des Lipidprofils von Eiern in einem Tier, das mit dem mindestens einen Traubenextrakt ergänzt wurde, ausgewählt aus der Gruppe bestehend aus oviparen und ovoviviparen Tieren, insbesondere Fischen und Geflügel.

2. Verwendung von mindestens einem Traubenextrakt nach Anspruch 1 zur Erhöhung des Gehalts an mindestens einer mehrfach ungesättigten Fettsäure in den Eiern des supplementierten Tieres, insbesondere des Gehalts an mindestens einer mehrfach ungesättigten Fettsäure aus der Familie der "Omega-3"-mehrfach-ungesättigten Fettsäuren, im Vergleich zu den Eiern des Tieres, das nicht mit dem mindestens einen Traubenextrakt supplementiert wurde.

3. Verwendung von mindestens einem Traubenextrakt nach dem vorstehenden Anspruch, **dadurch gekennzeichnet, dass** die mindestens eine mehrfach ungesättigte Fettsäure Alpha-Linolensäure und/oder Docosahexaensäure, insbesondere Docosahexaensäure, ist.

4. Verwendung von mindestens einem Traubenextrakt nach einem der Ansprüche 1 bis 3, **dadurch gekennzeichnet, dass** der Gehalt an mindestens einer mehrfach ungesättigten Fettsäure in den Eiern des supplementierten Tieres um mindestens 10 Gew.-%, vorzugsweise um mindestens 15 Gew.-% und am meisten bevorzugt um mindestens 20 Gew.-% erhöht ist, bezogen auf das Gewicht der mehrfach ungesättigten Fettsäuren in den Eiern des Tieres, das nicht mit dem mindestens einen Traubenextrakt supplementiert worden ist.

5. Verwendung von mindestens einem Traubenextrakt nach einem der Ansprüche 1 bis 4, worin der mindestens eine Traubenextrakt in einer Futtermittelzusammensetzung enthalten ist, worin die Futtermittelzusammensetzung ein Zusatzstoff, ein Futtermittel, ein Rohmaterial oder ein Ergänzungsfuttermittel ist.

6. Verwendung von mindestens einem Traubenextrakt nach Anspruch 5, **dadurch gekennzeichnet, dass** die Futtermittelzusammensetzung in fester Form vorliegt.

7. Verwendung von mindestens einem Traubenextrakt nach Anspruch 6, **dadurch gekennzeichnet, dass** die Menge an Traubenextrakt im Bereich von 10⁻⁴ Gew.-% bis 2 x 10⁻² Gew.-%, vorzugsweise von 5 x 10⁻⁴ Gew.-% bis 10⁻² Gew.-% liegt, bezogen auf das Gesamtgewicht des Trockenextrakts der Futtermittelzusammensetzung.

8. Verwendung von mindestens einem Traubenextrakt nach Anspruch 5, **dadurch gekennzeichnet, dass** die Futtermittelzusammensetzung in flüssiger Form vorliegt.

9. Verwendung von mindestens einem Traubenextrakt nach Anspruch 8, **dadurch gekennzeichnet, dass** die Menge an Traubenextrakt im Bereich von 10⁻⁴ Gew.-% bis 10⁻² Gew.-%, vorzugsweise von 2,5 x 10⁻⁴ Gew.-% bis 5 x 10⁻¹ Gew.-% liegt, bezogen auf das Gesamtgewicht der Futtermittelzusammensetzung.

10. Verwendung von mindestens einem Traubenextrakt nach einem der Ansprüche 1 bis 9, **dadurch gekennzeichnet, dass** die Eier für den menschlichen Verzehr oder zur Reproduktion bestimmt sind.

## Claims

1. A use of at least one grape extract, said at least one grape extract comprising:
a) from 50 to 95% by weight, preferably 65 to 85% total polyphenols, most preferably 80 to 85%, expressed as catechin equivalent, relative to the total weight of the extract,
b) from 30% to 90% by weight, preferably 50 to 70% proanthocyanidins, most preferably 60 to 70% relative to the total weight of the extract,
c) from 0.5 to 10% by weight, preferably 0.5 to 2%, most preferably 0.75 to 2% total anthocyanins and anthocyanidins relative to the total weight of the extract.
for the purpose of modifying the lipid profile of eggs in an animal that has been supplemented with said at least one grape extract, selected from the group consisting of oviparous and ovoviviparous animals, particularly fish and poultry.

2. The use of at least one grape extract according to claim 1 for increasing the level of at least one polyunsaturated fatty acid in the eggs of said supplemented animal, more particularly the level of at least one polyunsaturated fatty acid of the family of "omega-3" polyunsaturated fatty acids, relative to the eggs of said animal that has not been supplemented with said at least one grape extract.

3. The use of at least one grape extract according to the preceding claim, **characterized in that** said at least one polyunsaturated fatty acid is alpha-linolenic acid and/or docosahexaenoic acid, particularly docosahexaenoic acid.

4. The use of at least one grape extract according to any one of claims 1 to 3, **characterized in that** the level of at least one polyunsaturated fatty acid in the eggs of said supplemented animal is increased by at least 10%, preferably by at least 15%, and most preferably by at least 20% by weight relative to the weight of the polyunsaturated fatty acids in the eggs of said animal that has not been supplemented with said at least one grape extract.

5. The use of at least one grape extract according to any one of claims 1 to 4, said at least one grape extract being included in a feed composition, said feed composition being an additive, a feed, a raw material, or a complementary feed.

6. The use of at least one grape extract according to claim 5, **characterized in that** said feed composition is in solid form.

7. The use of at least one grape extract according to claim 6, **characterized in that** the amount of grape extract ranges from 10-4% to 2.10-2%, preferably from 5.10-4% to 10-2% by weight relative to the total weight of the dry extract of the feed composition.

8. The use of at least one grape extract according to claim 5, **characterized in that** the feed composition is in liquid form.

9. The use of at least one grape extract according to claim 8, **characterized in that** the amount of grape extract ranges from 10-4% to 10-2%, preferably from 2,5.10-4% to 5.10-1% by weight relative to the total weight of the feed composition.

10. The use of at least one grape extract according to any one of claims 1 to 9, **characterized in that** the eggs are intended for human consumption or for reproduction.
